(19)

Europäisches
Patentamt
European
Patent Office
Office européen
des brevets

(11) **EP 3 384 844 A1**

(12) **EUROPEAN PATENT APPLICATION**

(43) Date of publication:
**10.10.2018 Bulletin 2018/41**

(21) Application number: **17000576.3**

(22) Date of filing: **05.04.2017**

(51) Int Cl.:
*A61B 5/055* (2006.01)  *B06B 1/18* (2006.01)
*G01R 33/563* (2006.01)  *F03D 9/00* (2016.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Universität Heidelberg
69117 Heidelberg (DE)**

(72) Inventors:
• **Neumann, Wiebke
68167 Mannheim (DE)**
• **Zöllner, Frank Gerrit
68259 Mannheim (DE)**

(74) Representative: **Müller-Boré & Partner
Patentanwälte PartG mbB
Friedenheimer Brücke 21
80639 München (DE)**

(54) **A MECHANICAL ACTUATOR AND A METHOD FOR MAGNETIC RESONANCE
ELASTOGRAPHY USING CENTRIFUGAL FORCE**

(57)    The invention relates to a mechanical actuator
for Magnetic Resonance Elastography (MRE), a method
for inducing shear waves for Magnetic Resonance Elastography as well as respective system and method for
Magnetic Resonance Elastography using the principle of
centrifugal force for wave induction. The mechanical ac-
tuator comprises a passive driver including a rotational
turbine vibrator (10) having an eccentric weight (20), the
turbine vibrator (10) being powered by a fluid (e.g. com-
presses air or water), and an active driver (30) configured
to control the pressure of the fluid powering the turbine
vibrator (10).

Fig. 1A

**Description**

The present invention relates to a mechanical actuator for Magnetic Resonance Elastography (MRE), a method for inducing shear waves for Magnetic Resonance Elastography, as well as respective system and method for Magnetic Resonance Elastography using the principle of centrifugal force for wave induction.

Magnetic resonance elastography (MRE) is a technique for the quantification of tissue stiffness during MR examinations (see non-patent document 1). It can serve as an additional diagnostic tool to detect abnormal tissue stiffness and as a discriminator for benign or cancerous tissue (see non-patent document 2). During an MRE examination, an external source generates shear waves which propagate through the tissue. The sinusoidal displacement of the tissue is imaged with appropriate motion-encoding MR sequences. These sequences acquire so-called displacement fields which are then converted into stiffness maps (see non-patent document 3).

The quantification of tissue stiffness can be performed most efficiently in organs close to the body surface, due to the propagation characteristics of mechanical shear waves. A large size of the organ compared to the wave length is also beneficial (see non-patent document 4). In contrast, a reliable wave induction in deep-lying tissues such as the prostate (see non-patent documents 5 and 6), pancreas and kidney (see non-patent document 7) as well as myocardial tissue (see non-patent document 4) remains a major technical obstacle in MRE.

The MRE technique requires consistent methods for mechanical shear wave induction to the region of interest in the human body to reliably quantify elastic properties of soft tissues.

The most prominent systems for shear wave generation in previous MRE studies are acoustic driving systems that generate shear waves using a passive pneumatic drum driver (see non-patent documents 9-11) at frequencies in the range of 40 Hz to 200 Hz (see non-patent document 8). These pneumatic cushions are driven by varying acoustic pressure levels generated by an active audio device located outside the scanner room. The devices operate well at lower frequencies ($\approx 60$ Hz). However, at higher frequencies, the control of appropriate excitation amplitudes becomes problematic and additional power amplification is necessary to maintain a sufficiently large displacement range (see non-patent documents 4 and 12).

Other application-specific drivers were proposed using electromechanical coils (non-patent document 13) or piezoelectric drivers (see non-patent document 14), though these electromechanical actuators may introduce images artifacts, create a heat buildup typical for electromechanical drivers (non-patent document 10) or need to be actively shielded (see non-patent document 15).

Patent document 1 describes an air pressure driven ball vibrator for Magnetic Resonance Elastography that generates vibrations caused by a centrifugal force due to the rotation of a non-magnetic ball around the center point of the ball vibrator in a circumferential direction thereof. The ball vibrator is placed on a body surface to be examined, thereby generating a rotational motion on the body surface in a direction parallel to the body surface. However, although the ball vibrator can generate a large vibration, the parameters of the generated force (such as magnitude) are not easily adjustable. Further, the ball rotator is restricted to generating rotational motion in a direction parallel to the body surface.

List of non-patent documents:

[1] R. Muthupillai, D. J. Lomas, P. J. Rossman, J. F. Greenleaf, A. Manduca, and R. L. Ehman, "Magnetic resonance elastography by direct visualization of propagating acoustic strain waves," Science, vol. 269, pp. 1854-7, Sep 29 1995.

[2] R. S. Sahebjavaher, G. Nir, M. Honarvar, L. O. Gagnon, J. Ischia, E. C. Jones, et al., "MR elastography of prostate cancer: quantitative comparison with histopathology and repeatability of methods," NMR Biomed, vol. 28, pp. 124-39, Jan 2015.

[3] A. Manduca, T. E. Oliphant, M. A. Dresner, J. L. Mahowald, S. A. Kruse, E. Amromin, et al., "Magnetic resonance elastography: non- invasive mapping of tissue elasticity," Med Image Anal, vol. 5, pp. 237-54, Dec 2001.

[4] A. Arani, K. L. Glaser, S. P. Arunachalam, P. J. Rossman, D. S. Lake, J. D. Trzasko, et al., "In vivo, high-frequency three- dimensional cardiac MR elastography: Feasibility in normal volunteers," Magnetic Resonance in Medicine, vol. 77, pp. 351-360, 2017.

[5] R. S. Sahebjavaher, A. Baghani, M. Honarvar, R. Sinkus, and S. E. Salcudean, "Transperineal prostate MR elastography: initial in vivo results," Magn. Reson. Med., vol. 69, pp. 411-20, Feb 2013.

[6] R. Chopra, A. Arani, Y. Huang, M. Musquera, J. Wachsmuth, M. Bronskill, et al., "In vivo MR elastography of the prostate gland using a transurethral actuator," Magn. Reson. Med., vol. 62, pp. 665-71, Sep 2009.

[7] S. K. Venkatesh and R. L. Ehman, "Magnetic resonance elastography of abdomen," Abdom Imaging, vol. 40, pp. 745-59, Apr 2015.

[8] M. Yin, J. Chen, K. J. Glaser, J. A. Talwalkar, and R. L. Ehman, "Abdominal magnetic resonance elastography," Top Magn. Reson. Imaging, vol. 20, pp.

79-87, Apr 2009.

[9] O. Rouviere, M. Yin, M. A. Dresner, P. J. Rossman, L. J. Burgart, J. L. Fidler, et al., "MR elastography of the liver: preliminary results," Radiology, vol. 240, pp. 440-8, Aug 2006.

[10] M. Yin, J. A. Talwalkar, K. J. Glaser, A. Manduca, R. C. Grimm, P. J. Rossman, et al., "Assessment of hepatic fibrosis with magnetic resonance elastography," Clin Gastroenterol Hepatol, vol. 5, pp. 1207-1213 e2, Oct 2007.

[11] S. K. Venkatesh, M. Yin, J. F. Glockner, N. Takahashi, P. A. Araoz, J. A. Talwalkar, et al., "MR elastography of liver tumors: preliminary results," AJR Am J Roentgenol, vol. 190, pp. 1534-40, Jun 2008.

[12] P. Latta, M. L. Gruwel, P. Debergue, B. Matwiy, U. N. Sboto- Frankenstein, and B. Tomanek, "Convertible pneumatic actuator for magnetic resonance elastography of the brain," Magn. Reson. Imaging, vol. 29, pp. 147-52, Jan 2011.

[13] L. Huwart, F. Peeters, R. Sinkus, L. Annet, N. Salameh, L. C. terBeek, et al., "Liver fibrosis: non-invasive assessment with MR elastography," NMR Biomed, vol. 19, pp. 173-9, Apr 2006.

[14] K. Uffmann, C. Abicht, W. Grote, H. H. Quick, and M. E. Ladd, "Design of an MR-compatible piezoelectric actuator for MR elastography," Concepts in Magnetic Resonance, vol. 15, pp. 239-254, 2002.

[15] K. Uffmann and M. E. Ladd, "Actuation systems for MR elastography: design and applications," IEEE Eng. Med. Biol. Mag., vol. 27, pp. 28-34, May-Jun 2008.

List of patent documents:

Patent document 1: EP 2 499 970 A1.

In view of the above, it is an object of the invention to provide an improved mechanical actuator for Magnetic Resonance Elastography (MRE) and an improved method for mechanical wave generation or shear wave generation for Magnetic Resonance Elastography.

This object is solved by a mechanical actuator for magnetic resonance elastography according to claim 1, a system for magnetic resonance elastography according to claim 10, a method for inducing shear waves for magnetic resonance elastography according to claim 11, a method for magnetic resonance elastography according to claim 14, and a method for producing a mechanical actuator according to claim 15. Preferred embodiments are defined in the dependent claims.

According to an aspect, a novel mechanical actuator for inducing shear waves for Magnetic Resonance Elastography is proposed. The mechanical actuator uses the principle of centrifugal force for wave induction and comprises a passive driver including a rotational turbine vibrator powered by a fluid (i.e. a liquid or gas), such as for example water or compressed air (i.e. the rotational turbine vibrator is a pneumatic rotational turbine vibrator). The rotational turbine vibrator has at least one eccentric weight (also referred to as unbalance), which causes a rotary vibration. The at least one eccentric weight may be detachable or replaceable. It is thus possible to adjust the generated vibrational force.

The passive driver may be located inside a scanner room of a medical clinic where MRE examination and imaging is carried out and may be arranged/placed/fixed on a region of an elastic body (such as human or animal body) to be examined, in order to introduce shear weaves. The propagation of the shear waves is detected and imaged by a suitable magnetic resonance imaging (MRI) apparatus. Based on the propagation of the shear waves, qualitative and/or quantitative information regarding the elastic properties within the examined region may be obtained using known Magnetic Resonance Elastography methods. For example, qualitative and/or quantitative information of the elastic properties of various tissues within the examined region of a human or animal body may be obtained.

The mechanical actuator further includes an active driver configured to control the pressure of the fluid powering the turbine vibrator. The pressure of the fluid powering the rotational turbine vibrator may be in the range of 0 bar to 5 bar, preferably in the range of 0 bar to 2 bar. By controlling the pressure level of the fluid, the frequency and/or the amplitude of the generated vibration may be adjusted.

The generated force of the proposed mechanical actuator increases for higher actuation frequencies as opposed to conventionally used air cushions, in which the displacement amplitude decreases with increasing actuation frequency resulting in a smaller signal-to-noise ratio. More specifically, the generated force of the presented actuator increases with the increase of the rotational speed of the rotor of the rotational turbine vibrator, thus offering an elegant solution for sufficiently large wave actuation at higher frequencies and overcoming the dampening effects on the amplitude observed for the conventionally used pneumatic cushions at higher frequencies.

The materials used for the components of the passive driver may be any suitable MRE-compatible materials, preferably non-magnetic and/or non-metallic materials, such as polymers (for example polyamide, polyamide, polyurethanes, polypropylene, polycarbonate and acrylonitrile butadiene styrene), plastic, in particular thermoplastic, glass, non-ferromagnetic metals (such as brass) or a combination thereof. For any static part or component, non-magnetic materials are sufficient, for any rotating part or component (turbine rotor, unbalance, bearings), non-metallic materials are preferably used.

Preferably, at least a part of the components constituting the passive driver is produced by 3D printing. Any suitable 3D printing technique may be employed, for example selective laser sintering. In an example, all 3D printed components of the passive driver may be made of polyamide (PA). Thus, a low cost passive driver that is MR-safe and suits the range of wave inducing forces needed to generate appropriate shear waves in human or animal tissue may be obtained.

The rotational turbine vibrator comprises a housing and a turbine rotor arranged in the housing and being rotatably supported therein. The housing of the rotational turbine vibrator may be of a standard form or a customized form adapted to the region of the elastic body to be examined. The housing, the turbine rotor and the unbalance may be made of polymer, such as polyamide, polyamide, polyurethanes, polypropylene, polycarbonate and acrylonitrile butadiene styrene, and may be obtained by 3D printing technique.

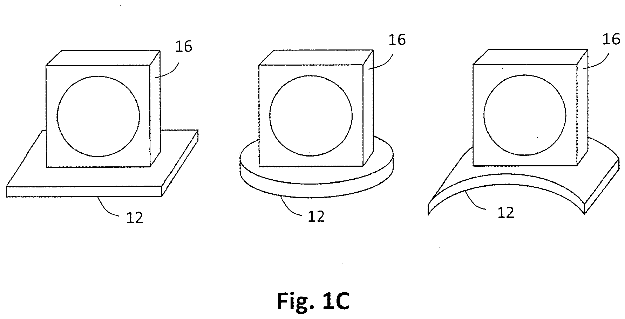

The passive driver may comprise further components (which may also be made of polymer and/or by 3D printing technique), such as for example a wave-applicator or adaptor configured to be placed on and/or fixed to the surface of a region of the elastic body to be examined. For example, the passive driver may comprise an adaptor plate connected or attached to the housing of the rotational turbine vibrator (for example via a suitable connector part) and configured to be positioned on and/or or fixed to a surface of a region of an elastic body (such as human or animal body) to be examined by a MRE technique. The adaptor plate and the housing of the rotational turbine vibrator may also be formed as single, unitary part. In other words, the adaptor plate may be "merged" with the rotational turbine vibrator. The vibrational force generated by the rotational turbine vibrator is transferred through the wave-applicator/adaptor (for example through the adaptor plate) to the region to be examined, thereby generating a substantially sinusoidal mechanical wave in the elastic body. The propagation of the sinusoidal mechanical wave in the elastic body may be detected and evaluated using known MRI te chniques. The propagation velocity of the generated wave provides qualitative and quantitative information of the differences in the elasticity in different areas of the examined region of the elastic body, such as the differences in the elasticity of different tissues in the examined region of a human or animal body. The adaptor plate may be a standard adaptor plate or a customized adaptor plate, for example an adaptor plate adapted to the region of the elastic body to be examined (e.g. Fig. 1 C).

Further, the rotational turbine vibrator may comprise an inlet (for example constituted by a pneumatic fitting) made of non-ferromagnetic metal (such as brass) or other non-magnetic material. The rotational turbine vibrator may also comprise a sound absorber (for example made of plastic), serving as an outlet of the fluid from the rotational turbine vibrator. The turbine rotor may be rotatably supported in the housing by means of bearings, for example substantially spherical rolling bearings made of thermoplastic and glass.

Preferably, the generated centrifugal force is in the range of 0.1 N to 50 N. The generated centrifugal force may be varied or adapted to the region of the elastic body to be examined. The generated centrifugal force may be varied depending on the age of the subject to be examined (child, adolescent, grown-up), the type and position of the organ to be examined, the type and/or and strength of the fixation of the passive driver to the elastic body, etc.

The weight of the unbalance and/or the size of the turbine vibrator and in particular the ratio of the weight of the unbalance to the size of the turbine vibrator may be appropriately selected to achieve appropriately large but tolerable wave actuation for a given nominal frequency. For example the weight of the unbalance may be selected to be in the range of 1 g to 30 g, preferably in the range of 3 g to 15 g. Further, the distance of the center of mass of the unbalance to the rotational center of the turbine rotor of the rotational turbine vibrator may be in the range of 1 mm to 15 mm, preferably in the range of 3 mm to 10 mm.

Preferably, the frequency and/or amplitude of the vibration generated by the rotational turbine vibrator may be varied stepwise or continuously within predetermined ranges. For example, the frequency of generated vibration (vibrational force) may be in the range 10 Hz to 400 Hz, preferably in the range of 30 Hz to 120 Hz. The amplitude of the generated vibration may be in the range of 0.5 mm to 5 mm, preferably in the range of 1 mm to 3 mm.

The active driver controls the parameters of the vibration/generated vibrational force by controlling the pressure of the fluid powering the rotational turbine vibrator. The active driver may for example comprise a proportional pressure regulator (for example a voltage-controlled pressure regulator that may include a valve, a controllable resistance and a DC-regulator) configured to control the pressure of the fluid (e.g. water, compressed air or other gas) powering the rotational turbine vibrator by adjusting a control voltage. For example, the magnitude of the control voltage may be adjusted within the range of 0 V to 10 V, preferably in the range of 0 V to 6 V, further preferably in the range of 0.1 V to 3 V. Depending on the type of the employed valve other voltage levels are possible, for example higher or lower voltage levels. The stepwise or continuous regulation of the control voltage may be updated within the range of 0 Hz to 1000 Hz, preferably in the range of 10 Hz to 100 Hz. In other words, the regulation frequency or rate of the control voltage may be in the range of 0 Hz to 1000 Hz, preferably in the range of 1 Hz to 100 Hz. The above values may vary, depending on the type of regulator, the employed valve or other parameters of the system.

In order to ensure safety, several safety features may be implemented individually or in combination. For example, the proportional pressure regulator may be configured to stop the inflow of fluid in the passive driver when no control voltage is applied and/or in case of power loss. In other words, the passive driver may be designed to be normally "closed", meaning that if no control voltage is applied to the system, or in case of power loss, a valve of the pressure regulator closes and no fluid is fed into the turbine.

Further, the passive driver may be configured to stop the inflow of compressed fluid to the passive driver and upon user input, for example via a suitable (graphical) user interface, indicating that the inflow of fluid in the passive driver should be stopped. For example, the graphical user interface (GUI) may be provided with an emergency stop button, slide or any other suitable icon. Upon receiving user input via the graphical user interface (for example via the emergency stop button), the passive driver may be configured to instantly set the control voltage to 0 V and to stop the output of fluid.

The active driver may also comprise a manual valve configured to stop the inflow of fluid (e.g. air or other gas) in the passive driver when operated by a user. The manual valve may be positioned between the source of the fluid (for example an in-house pressure hose supplying compressed air) and the proportional pressure regulator. This allows an operator to manually stop the inflow of fluid in the driver system.

Further, the proportional pressure regulator may be configured to increase the control voltage gradually or stepwise during a start-up phase until a predetermined value is reached. Thus, a constant communication with the patient may be maintained to ensure that the induced vibration level is tolerable to the patient.

In an example, the passive driver comprises a plurality of rotational turbine vibrators connected in series through a common axle (common drive shaft). In this example the active driver synchronously drives the turbine vibrators connected in series. The use of multiple synchronously driven mechanical actuators enables wave actuation at multiple locations on the body with synchronized mechanicals waves, thereby increasing the spatial resolution of the obtained elastograms.

According to another aspect, there is provided a magnetic resonance elastography system comprising a magnetic resonance imaging (MRI) apparatus and the mechanical actuator according to any one of the examples described above. The MRI apparatus may be any conventional MRI apparatus/scanner. The MRI imaging apparatus may for example comprise an image acquisition device and an image evaluation device including for example a suitably programmed processing unit (for example a general purpose or a dedicated processor).

Still another aspect of the invention relates to a novel method for sinusoidal mechanical wave generation based on the principle of centrifugal force employing the mechanical actuator according to any one of the above examples. The method uses a fluid powered turbine vibrator having an eccentric weight (unbalance), to generate a rotary vibration. The generated centrifugal force has an amplitude depending on the driving frequency as well as on the weight and dimensions of the unbalance. The frequency itself can be chosen arbitrarily via the applied air pressure level.

In particular, a method for inducing vibration in an elastic body for magnetic resonance elastography may comprise:

providing a mechanical actuator according to any of the above described examples;
positioning the passive driver of the mechanical actuator on a region of the elastic body to be examined, controlling, by the active driver of the mechanical actuator, the pressure of the fluid powering the rotational turbine vibrator, thereby generating, by the rotational turbine vibrator, a vibration force having a predetermined frequency and/or magnitude.

The passive driver of the mechanical actuator may comprise a plurality of rotational turbine vibrators connected in series through a common axle and the method may comprise positioning the plurality of rotational turbine vibrators on a plurality of locations within the region to be examined and controlling by the active driver the pressure of the fluid powering each of the plurality of rotation turbine vibrators.

As explained above, the controlling of the pressure of the fluid (e.g. water, compressed air or other gas or liquid) may comprise adjusting a control voltage of a proportional pressure regulator of the active driver. The magnitude of the control voltage may for example be adjusted within the range of 0 V to 10 V, preferably in the range of 0.1 V to 3 V The stepwise or continuous regulation of the control voltage may be updated within the range of 0 Hz to 1000 Hz, preferably in the range of 1 Hz to 100 Hz. In other words, the regulation frequency or regulation rate of the control voltage may be in the range of 0 Hz to 1000 Hz, preferably in the range of 1 Hz to 100 Hz. The above values may vary, depending on the type of regulator, employed valve or other parameters of the system.

Further as explained above, several safety features may be implemented alone or in combinations. For example, the control voltage may be adjusted to increase gradually or stepwise up to a predetermined value during a start-up phase. The inflow of fluid in the passive driver may be stopped when no control voltage is applied and/or in case of power loss and/or upon user input. The user input may be received through a suitable graphical user interface (GUI) or by manually operating a manual valve of the active driver.

Still another aspect relates to a method for magnetic resonance elastography comprising:

inducing shear waves in region of an elastic body to be examined according to the above described method;
imaging the propagation of the induced shear waves, thereby obtaining at least one wave image; and obtaining elasticity data of the region of the elastic body to be examined based on the at least one wave image.

The at least one image of the propagating shear wave may be obtained by a conventional magnetic resonance imaging (MRI) apparatus. The obtaining of elasticity data of the region of the elastic body to be examined may comprise converting the at least one wave image into an elastogram of the region to be examined, for example by application of suitable algorithms, such as inversion algorithms.

According to an example, the shear waves may be induced synchronously at a plurality of locations within the region of the elastic body to be examined. The propagation of the synchronously induced shear waves may be imaged, thereby obtaining at least one wave image. Based on the obtained wave image(s), elasticity data of the region of the elastic body to be examined. The synchronous actuation at a plurality of locations within the region to be examined increases the spatial resolution of the obtained elastograms, enabling thereby detection of smaller inclusions (for example smaller tumors) as compared to the case where only one rotational turbine vibrator is used.

Yet another aspect of the invention relates to a method for producing a mechanical actuator for Magnetic Resonance Elastography according to any one of the above described examples, comprising producing the housing, the turbine rotor and the unbalance and/or an adaptor plate (which may be a customized adaptor plate) by 3D printing or other suitable methods, such as molding, machining, etc.. The method may comprise producing the housing, the turbine rotor, the unbalance and/or the adaptor plate by 3D printing and connecting the adaptor plate to the housing (for example by a suitable connector portion that may be a part of the housing and that may be also produced by 3D printing). Thus, inexpensive mechanical actuator for Magnetic Resonance Elastography may be obtained.

The method may further comprise configuring or adapting the mechanical actuator and/or at least one component thereof based on at least one property or characteristic of the elastic body and/or the region of the elastic body to be examined. The at least one component may be any one of the housing, the turbine rotor, the unbalance, the adaptor plate, and/or other components of the mechanical actuator.

The configuring or adapting the mechanical actuator and/or at least one component thereof may comprise determining or varying one or more characteristics or parameters of the mechanical actuator and/or at least one component thereof, including for example the nominal force of the generated vibration, dimensions and/or form of the housing, weight and/or arrangement of the unbalance, form and/or dimensions of the adaptor plate, and/or other parameters.

The at least one property or characteristic of the elastic body to be examined and/or the region thereof may include any one of a type, size, material, topography and/or other parameters of the elastic body and/or the region of the elastic body to be examined. For medical applications the one or more characteristics of the mechanical actuator may be determined based on the type, size, topography and/or form and/or localization of the examined organ(s), age of the patient, maximum tolerable force, etc. The at least one property or characteristic of the elastic body and/or the region thereof may be obtained by a measurement (such a measurement of the size and/or topography of the region of the elastic body to be examined) and/or from a database, for example a database storing a property or properties of a standard elastic bodies and/or regions thereof.

Based on the determined one or more parameters or characteristics of the mechanical actuator and/or at least one component thereof, 3D construction data (for example in the form of CAD data or program instructions) may be generated and used to produce the mechanical actuator and/or the at least one component thereof. The 3D construction data may be for example 3D printing data for the 3D printed components of the mechanical actuator (such as the housing, the turbine rotor, and/or the adaptor plate). The respective components are subsequently 3D printed using the 3D printing data and assembled. Thus, it is possible to easily and efficiently obtain a customized, yet inexpensive mechanical actuator for Magnetic Resonance Elastography, for example a mechanical actuator customized for specific patient and/or specific organ.

In an example, the size and/or topography of an adaptor plate may be customized. The customizing may include configuring or adapting the adaptor plate to conform to the size and/or topography of the region of the elastic body to be examined, thereby producing 3D adaptor plate printing data. The customized adaptor plate may be connected to the housing of the mechanical actuator, for example by a connector/connector portion (which may be a part of the housing). The housing may be a standardized housing or a customized housing (i.e. a housing configured or adapted based on at least one property of the region of the elastic body to be examined). Preferably, the housing is also produced by 3D printing.

The above and other objects, features and advantages of the present invention will become more apparent upon reading of the following detailed description of preferred embodiments and accompanying drawings. It should be understood that even though embodiments are separately described, single features thereof may be combined to additional embodiments.

Brief description of the drawings:

Fig. 1A is a plan view showing the structure and components of the pneumatic turbine;
**Fig. 1 B** is a side view showing the structure and components of the pneumatic turbine;
**Fig. 1 C** shows schmatically adaptor plates of varying designs;
**Fig. 2** shows schematically a MRE system including a pneumatic turbine;
**Fig. 3** shows the force generated by the pneumatic turbine as a function of the frequency of vibration;
**Fig. 4** shows a coronal view of a cylindrically shaped gelatin phantom;
**Fig. 5** illustrates the generation of a given force with turbines having different geometries;
**Fig. 6** illustrates the generation of a force in a direction parallel to the body surface (left hand side) and in a direction perpendicular to the body surface (right hand side);
**Fig. 7** shows schematically an exemplary passive driver including a plurality of turbine vibrators.

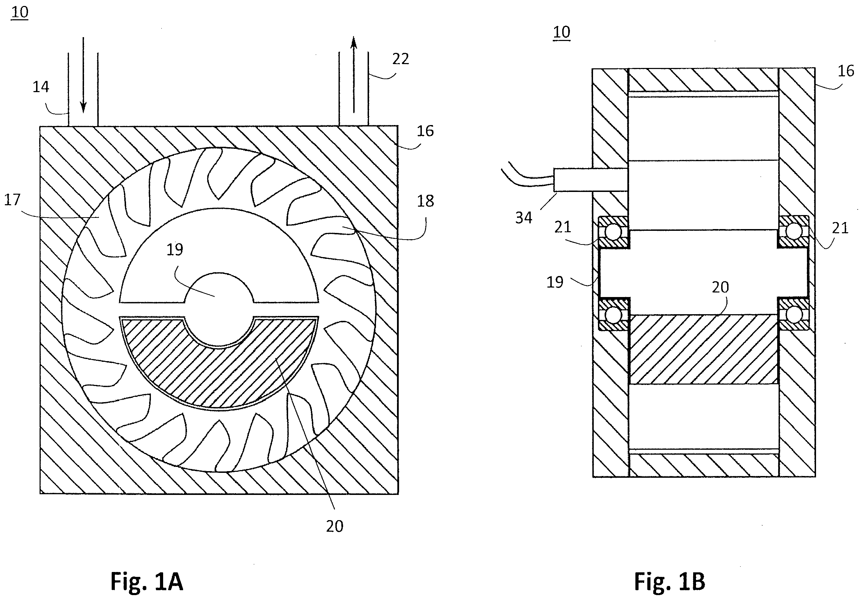

The mechanical actuator according to an example comprises a passive driver including or constituted by a pneumatic turbine (also referred to as a pneumatic vibrator or rotational pneumatic vibrator) 10 powered by compressed air, as illustrated in Fig. 1A and 1B. In this examples, compresses air is used, however other gases or liquids may be used for driving the pneumatic turbine 10.

The pneumatic turbine comprises a housing 16 having an interior rotor chamber 17, which may have a substantially cylindrical form. A turbine rotor 18 having a central driving shaft (axle) 19 is arranged within the interior rotor chamber 17. The rotor 18 is rotatably supported within the chamber by means of bearings, for example spherical rolling bearings 21. E.g. compressed air is supplied to the pneumatic turbine 10 via an air inlet 14 and exits the pneumatic turbine 10 through a sound absorber 22. The input and output air streams are directed to and out of the rotor chamber 17 via conduits formed in the housing (not shown in Fig. 1A and 1B). In this example a pneumatic fitting (for example a pneumatic fitting M022A0605 of Norgren GmbH, Germany) serves as the air inlet 14 for inflow of compressed air into the turbine. The sound absorber 22, which serves as an air outlet, may be made of plastic (for example M/1545, Norgren GmbH, Germany).

The turbine rotor 18 is provided with an eccentric weight (unbalance) 20 arranged in an opening thereof between the axle 19 and the rotor blades, which causes a rotary vibration. Preferably, the unbalance 20 is exchangeable, which enables adapting the turbine 10 for different levels of force generation. The unbalance 20 may be made of polyamide or other suitable polymers. The unbalance may for example have a half-cylindrical shape having for example a height h in the range of 2 mm to 50 mm, more specifically in the range of 5 mm to 40 mm; and a weight m in the range of 2 g to 30 g, more specifically in the range of 3 g to 15 g. The distance $r_{ecc}$

of the center of mass of the unbalance 20 to the rotational center of the turbine rotor 18 may be for example in the range of 1 mm to 15 mm, more specifically in the range of 3 mm to 10 mm. Thus, for vibration (actuation) frequencies ranging from 10 Hz to 400 Hz, more specifically in the range of 30 Hz to 120 Hz the generated forces may be in the range of 0.01 N to 150 N more specifically between 0.01 N and 10 N.

The geometry of the housing the turbine may be designed in such a way that it encloses the turbine rotor and provides a connective part at the bottom such that an adaptor plate can be fixed to the housing. The turbine rotor may have a diameter in the range of 10 mm to 100 mm, more specifically in the range of 20 mm to 50 mm. The length of the turbine may be in the range of 10 mm to 100 mm, more specifically in the range of 20 mm to 50 mm. It may also be possible to design a rotor, where the length of the cylindrical shape is larger compared to its diameter. The number of rotor blades may range from 6 to 50, more specifically in the range of 10 to 20 blades. The blades may be distributed symmetrically around the rotational center.

As explained above, the pneumatic turbine 10 is a rotational pneumatic vibrator creating a centrifugal force by the eccentric weight (unbalance) 20 within the turbine rotor 18. The generated force *F* depends on the weight of the unbalance $m_{ecc}$, the distance $r_{ecc}$ of the center of mass of the unbalance to the rotational center of the turbine 10 and more specifically to the rotational center of the turbine rotor 18 as well as the rotational speed $\omega$ of the turbine rotor 18. The generated force F can be computed via the formula:

$$F = m_{ecc} r_{écc} \omega^2$$

Consequently, a larger force can be generated by one or more of 1) using materials with higher densities for the unbalance, 2) changing the geometry such that the distance of the unbalance's center of mass to the rotational center increases, 3) enlarging the volume of the unbalance, and 4) increasing the frequency of the turbine. For sufficiently large but tolerable wave actuation, the ratio of the weight of unbalance to the size of turbine may be optimized.

Accordingly, a pre-set force $F_n$ and wave actuation frequency $f_n$ may be generated through the variation of a number of parameters (individually or in combination).

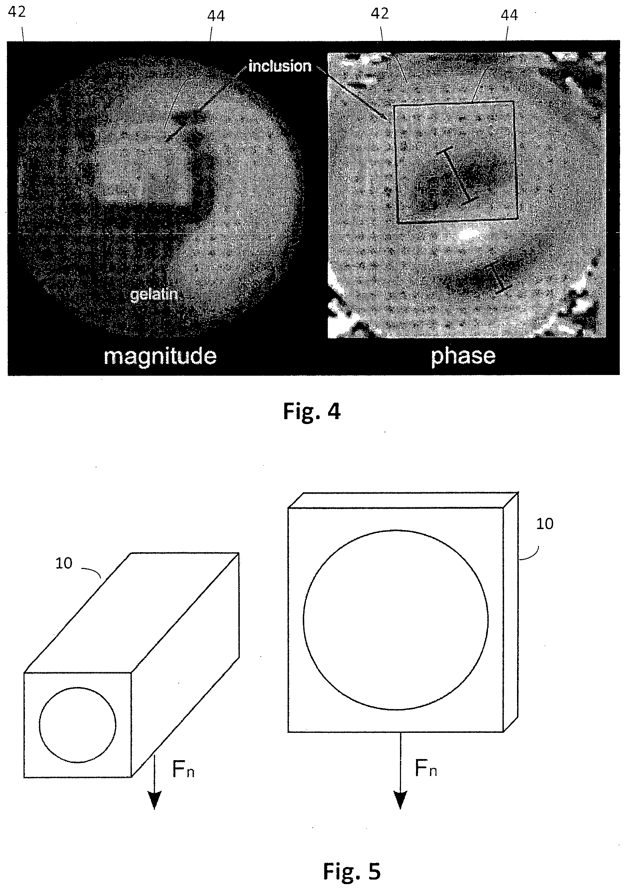

For example, a pre-set (nominal) force may be generated with housings having different geometries. As shown in **Fig. 5**, a turbine vibrator with a small diameter but large cylindrical height can exert the same force as a turbine vibrator with a large diameter but small cylindrical height. Accordingly, for a given force, the size and geometry of the housing and thus the size and geometry of the turbine rotor can be adapted to the outer geometric restrictions on the patient and in the MR-bore. In particular, the housing geometry may be selected such as to fit in holes of body, flex or other coils (vendor independent) and still exert the same nominal force. Examples include a housing with a constant width and variable depth for flat designs; a housing with variable width and small depths for small but high designs, etc. In contrast, the geometry of a ball vibrator disclosed in above mentioned patent document 1 is highly dependent on the radius of the ball and increases proportionally in height and width and depth with increasing ball size.

Further, different forces may be generated by employing unbalances having different weight and/or arrangement. The turbine vibrator may be configured such that the unbalance is removable and replaceable with another unbalance, for example an unbalance being made of the same material, but having a different volume (for example by changing the cylindrical height of the unbalance) or by an unbalance having the same geometry, but being made of a material with higher or lower density. The turbine rotor may for example exhibit one or more unbalance accommodating portions (e.g. bores or recesses), wherein the unbalance is releasably accommodated in the respective unbalance accommodating portions.

At least some of the parts of the pneumatic turbine 10, such as the housing 16 and/or the turbine rotor 18 and/or the unbalance 20 may be 3D printed. The 3D printed parts can be designed with a suitable CAD software (for example Autodesk Inventor, Autodesk GmbH, Germany) and may be made by selective laser sintering (for example using 3D printers of Materialise GmbH, Germany).

Since the pneumatic turbine 10 is located within a scanner room where MRI imaging is carried out, it is exposed to high magnetic fields, for example $\geq 1$ T, in particular about 1.5 to 3 T. Thus, preferably no magnetic components are used, i.e. the components constituting the pneumatic turbine 10 (as wells as the adaptor plate 12) are preferably made of non-magnetic and/or non-metallic materials, such as plastic, glass, non-ferromagnetic metals (such as brass) and/or combinations thereof. For any static part, non-magnetic materials are sufficient. For any rotating part (such as turbine, unbalance, bearings) the material should be non-metallic as well.

For example polyamide (PA) may be used for all 3D printed parts. Other non-magnetic material such as for example polyurethanes (PU), polypropylene (PP), polycarbonate (PC) acrylonitrile butadiene styrene (ABS) or other (thermoplastic) polymers may be used. The spherical rolling bearings may be made of polyoxymethylene (a thermoplastic) and glass (based on DIN 625-626). The sound absorber 22 may be made of plastic. In this example, the pneumatic fitting that serves as the inlet 14 for compressed air into the turbine is the only metal-containing part. However, the pneumatic fitting is made of non-magnetic brass and does not experience forces during MR measurements. Other suitable non-magnetic materials may be used, such as for example polymers (PA, PU, PP, PC, ABS, epoxy resins) or non-magnetic ceramics.

At the bottom, the pneumatic turbine 10 and more specifically the housing 16 can be attached to an adaptor plate 12 of varying geometries. **Fig. 1C** shows schematically adaptor plates 12 of varying designs that can be attached to the housing 16. For example, the housing 16 may include a connector part for connecting the housing to adaptor plates with different geometries. The adaptor plate 12 is configured to be placed on the surface of the volume/region of interest of a human or animal body. The size and/or geometry of the turbine 10 and/or the surface geometry of the adaptor plate 12 may be optimized for in-vivo imaging. The adaptor plate may be optimized to fit the geometry of the location on the body surface for wave actuation. For example, if the examined body part is femoral, slightly curved adaptor plates may used. If the examined body part is an abdomen, the adaptor plate may have a substantially plane surface. Circular plates with a diameter for example in the range of 20 mm to 200 mm, more specifically in the range of 50 mm to 150 mm, or rectangular plane or bend plates with an area in the range of 4 $cm^2$ to 225 $cm^2$, more specifically in the range of 9 $cm^2$ to 100 $cm^2$ may be used.

The connecting part of the turbine 10 to the adaptor plate 12 may be designed to fit in one of the pockets of a commercially available body coil 24 for fixation (for example Body coil 18 from Siemens, Erlangen, Germany) and, therefore, does not need additional mounting support. However, an additional mounting support may also be provided.

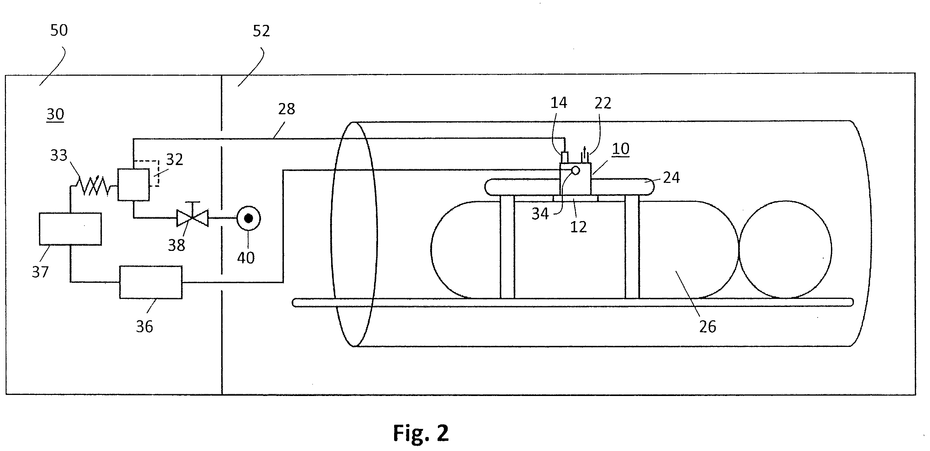

**Fig. 2** shows schematically a MRE system comprising a mechanical actuator using centrifugal force. The mechanical actuator comprises a passive driver including a pneumatic turbine 10 (which may be the above described pneumatic turbine) and an active driver 30. The pneumatic turbine 10 is positioned (via an adaptor plate 12) on the surface of the region of interest of a patient 26. For example, the pneumatic turbine may be arranged under or within one of the pockets of a body coil 24 for fixing the patient 26.

The air inlet 14 of the pneumatic turbine 10 is connected by a conduit (pipe) 28 and via a pressure regulator 32 to a pressure hose 40. The pressure hose is typically available in all scanner rooms of a medical clinic in accordance with the norm DIN 13260-2. The pressure hose 40 typically supplies compressed air with a nominal pressure of $p_{hose}$ = 5 bar (5 * $10^5$ Pascal).

All magnetic (i.e. MR-unsafe) and active electronic parts of the MRE system are located in a control room 50 (i.e. are placed outside of the scanner room 52). These parts comprise the active driver unit/system for regulating the pressure of the e.g. compressed air powering the turbine such that a specific actuation frequency is achieved.

The active driver unit (active driver) 30 comprises a proportional pressure regulator 32 having a controllable resistance 33 (such as for example VPPM-6L-L-G18-0L6H-V1N-S1, Festo Vertrieb GmbH & Co. Kg, Germany) connected to the in-house pressure hose 40 and the air inlet 14 via the conduit 28. The compressed air from the pressure hose 40 is fed to the proportional pressure regulator 32, which regulates the output pressure of the compressed air via a control voltage. The proportional pressure regulator 32 sets the output air pressure by adjusting the control voltage, for example by adjusting the control voltage within a range of 0 V to 10 V (corresponding to minimal/maximum pressure output). During start up, the control voltage, and according the output pressure, may increase gradually or stepwise until the nominal frequency of the pneumatic turbine 10 is reached.

To ensure controlled output of fluid, several safety features may be implemented alone or in combination. For example, the pressure regulator 32 may be designed such as to be 'normally closed', meaning that if no control voltage is applied to the system, or in case of a power loss, the valve of the pressure regulator closes and no fluid is fed into the turbine. Further, the control voltage may be controlled to increase stepwise during start up, such that a constant communication with the patient can be maintained to ensure that the induced vibration level is tolerable for the patient. Thirdly, an emergency stop button (for example an emergency stop button on a graphical user interface 37 of the active driver) can be implemented, configured to instantly set the control voltage to 0 V and stop the output of the fluid. Lastly, a manual stop valve 38 may be placed between the in-house pressure hose 40 and the pressure regulator 32. This allows an operator to manually stop the inflow of the fluid in the driver system.

A probe/sensor 34 providing a feedback on the actuation frequency of the pneumatic turbine 10, such as an MR-safe fiber-optic sensor (for example WLL180T, Sick AG, Germany), may be attached to the housing of the turbine 10 to provide feedback on the actuation frequency to the pressure regulator 32. The optical signal(s) detected by the sensor 34 is/are converted into electrical signal(s) indicative of the rotational speed of the turbine by a suitable electro-optical interface 36. The measured rotational speed of the turbine may be evaluated, for example by using a suitable electronic circuitry and may be fed into a feedback loop that regulates the control voltage, i.e. the output pressure, of the proportional pressure regulator 32. The electronic circuitry may be for example a general-purpose or dedicated electronic circuitry configured to implement a software package, such as Labview, NI USB-6525, National Instruments Germany GmbH, Germany, for processing the detected actuation frequency. The electronic circuitry may be connected to a user interface 37.

The fiber-optic sensor 34 may be configured to detect one or more signals per rotation of the turbine. For example, optical markers may be placed on one or more of the rotor blades. The optical marker(s) is/are detected by the optical sensor 34 during the turbine rotation, thereby producing the one or more signals. The detection of more than one optical signal per rotation increases the temporal resolution and/or shortens the measurement intervals. In contrast, the ball vibrator disclosed in the above mentioned Patent Document 1 is capable of detection of only one signal per rotation. This means, that the temporal resolution is quite restricted. One has to measure for a long time to receive a sufficiently accurate temporal resolution of the actuation frequency (with little information about fluctuation in the signal frequency).

The number of rotations may be counted over a time interval of 1000 ms and subsequently the frequency is calculated, updated in the active driver system and displayed in the user interface 37. During the technical evaluation of the actuator, nominal frequencies may be set between 15 Hz and 60 Hz with a step width of 5 Hz for a time interval of 30 seconds each. The stability of each frequency may be recorded and evaluated with suitable software programs, for example Matlab of The MathWorks, Inc. USA.

The force generated by the pneumatic turbine 10 may be measured for example by a load detector (not shown in Fig. 2), for example a precision miniature load cell such as 9206-V0001 of Burster Präzisionstechnik GmbH & Co. KG, Germany, connected to the bottom of the turbine 10.

The feasibility of using the mechanical actuator for wave actuation during MRE was experimentally confirmed on a cylindrically shaped phantom 42 having elasticity similar to that of soft tissue. This was achieved by adding 7.5 % gelatin (240 bloom) to 1000 ml distilled water. A cubical inclusion 44 (side length 50 mm) made of distilled water with 1.0 % agarose was placed in the center of the phantom 42. A concentration of 0.03 % sodium azide acting as a preservative was added to both materials. All chemicals were acquired from Carl Roth GmbH & Co. KG (Germany). The gelatin-agarose phantom was placed in a 3 T whole-body scanner (Magnetom Skyra, Siemens, Germany). Imaging was performed using an eight- channel receive-only phase-based array coil. A gradient- echo based sequence was employed (TE/TR = 20/50 ms, matrix size= 256x60, FOV = 450 mm, slice thickness = 5 mm).

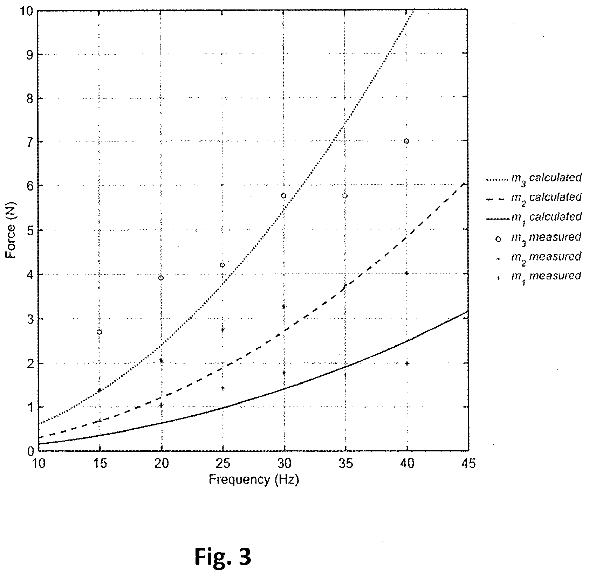

Three 3D printed unbalances made of polyamide with varying cylinder heights ($h_1$ = 9.5 mm, $h_2$ = 19.0 mm, $h_3$ = 38.0 mm) were designed to examine the influence of varying eccentric weights upon the generated forces. The weights of the 3D-printed unbalances were $m_1$ = 4.5 g, $m_2$ = 8.7 g and $m_3$ = 17.4 g, respectively. The calculated distance of the center of mass of the unbalance to the rotational center of the turbine was $r_{ecc}$ = 8.8 mm. Thus, for frequencies ranging from 15 Hz to 60 Hz, the theoretically generated forces are expected to be between 0.41 N to 1.6 N for the smallest and 6.50 N to 26.0 N for the largest unbalance (see **Fig. 3**).

An initial response behavior of the turbine was triggered at 10 Hz. Nominal frequencies were set between 15 Hz and 60 Hz (step width of 5 Hz) during the feasibility study and were kept stable within a range always smaller than ± 0.3 Hz. As predicted, the generated forces depended on the weight of the unbalance within the turbine and increased from 0.67 N to 3.09 N (4.5 g) and from 2.7 N to 7.77 N (17.4 g) in the analyzed frequency range of 15 Hz to 60 Hz (see **Fig. 3**). The experimentally obtained force levels matched the theoretical values closely in the lower frequency range, with any observed deviations probably caused by increased friction and additional weight of the turbine. The proposed pneumatic vibrator generated forces that were sufficiently large to penetrate the phantom entirely during the feasibility study. Thus, shear waves propagated through the entire volume of the phantom 42.

Magnitude and phase images were obtained at a frequency of 60 Hz generated by the proposed pneumatic vibrator. **Fig. 4** shows a coronal slice of the cylindrically shaped gelatin phantom acquired with MRI. The left hand side of Fig. 4 shows a magnitude image of the phantom with the propagating shear waves, in which the inclusion is indicated with an arrow. The right hand side of Fig. 4 shows a phase image acquired with a gradient-echo based sequence. The spatial length of the same phase angle is larger within the inclusion compared to the phase angle in the background material (black markers). The stiffer inclusion 44 could be detected by an increased wave length compared to the wave length in the background material, as indicated in **Fig. 4.** The passive driver did not produce any artifacts in the acquired MR images.

Thus, the proposed mechanical actuator for MRE has advantages over conventionally applied pneumatic cushions, since using centrifugal forces rather than acoustic pressure levels, it is possible to achieve sufficiently large wave actuation at higher frequencies as compared to air cushions, where the amplitude of sound pressure waves dampens with increasing frequencies. The actuation frequency can be for example regulated smoothly between 10 Hz and 150 Hz and between 10 Hz and 70 Hz for unbalances with a weight of 4.5 g and 17.4 g, respectively. The maximum applicable frequency is only restricted by the available in-house air pressure system but well within the range of currently applied frequencies of wave actuation for MRE imaging.

The driver is easy to set up and can be incorporated within existing equipment in a medical clinic. The passive driver is MR- safe and does not interfere with the imaging procedure. The design is adaptable and may be easily reproduced through low-cost 3D- printing.

In operation, the mechanical actuator according to any of the above described examples may be provided and set-up as described above. An exemplary method for inducing vibration in an elastic body for magnetic resonance elastography comprises positioning the passive driver on a region of the elastic body (for example a human or animal body) to be examined and controlling, by the active driver, the pressure of the fluid powering the rotational turbine vibrator. The rotational turbine vibrator generates thereby a vibration force having a predetermined frequency and/or magnitude, which may be controlled by the active driver as described above. The waves propagating through the region to be examined may be detected and evaluated by using conventional MRI/MRE apparatuses and methods. Based on the detected waves, qualitative and/or quantitative data of the elastic properties of various areas of the examined region (such as for example the elastic properties of the various tissues in the examined region) may be determined. On the basis of the information, the areas/tissues within the examined region may be discriminated.

Further as explained above, several safety features may be implemented alone or in combinations.

As explained above, the proposed mechanical actuator for Magnetic Resonance Elastography and the respective methods according to embodiments of the invention may have one or more of the following features and/or advantages:

a) Modular set up, in particular:

◦ Easily adaptable and customizable through simple exchange of components, such as unbalances, adaptor plates, etc.;
◦ Cost efficient, since one turbine can be equipped with variable eccentric weights for different levels of force generation and variable adaptor plates;
◦ Simple and fast production of additional parts (i.e. unbalances, adaptor plates) through 3D printing;

b) Variable geometry of the adaptor plates or housing of the rotational turbine vibrator

◦ Depending on the location to be imaged (thigh, abdomen, liver, head) and patient size (pediatric, small and big adult patient) adaptor plates and housings with varying surface geometries (e.g. circular, rectangular, irregular shaped; plan or bend; see for example Fig. 1 C) can be chosen to be optimally attached to the particular body surface;

c) Multi-parametrical configuration of the wave actuation system for a nominal exerted force/mechanical wave amplitude at body surface

◦ As explained above a pre-set force $F_n$ and wave actuation frequency $f_n$ may be generated through the variation of a number of parameters (individually or in combination), including:

▪ variable housing and thus turbine geometry: For example, the size of the housing can be adapted to outer geometric restrictions on the patient and in the MR-bore;

■ variable weight of the unbalances: For example unbalances made of the same material, but with different volumes (through for example the change in the cylindrical height) or unbalances having the same geometry, but being made of materials with different densities may be employed;

d) Variable direction of force generation

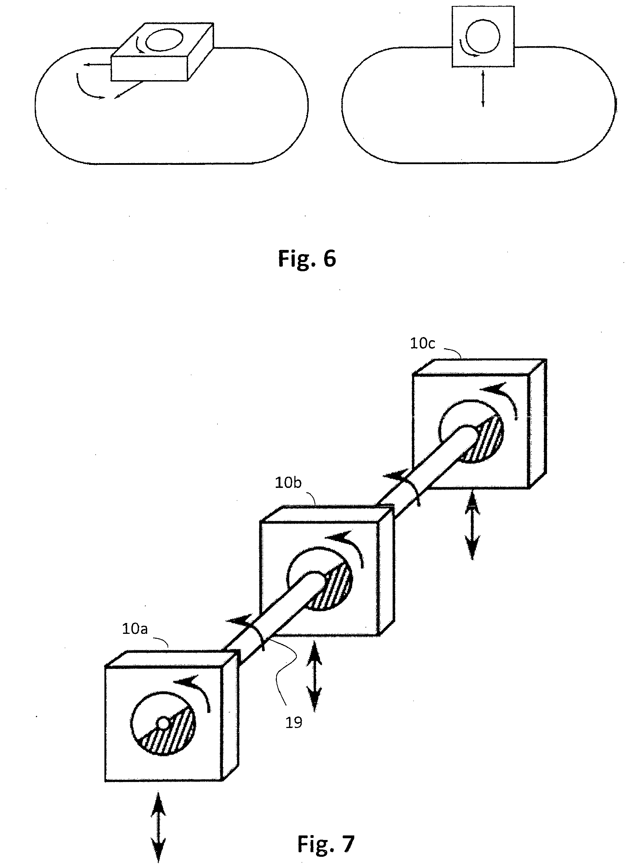

◦ The turbine vibrator can be used (similar to the ball vibrator disclosed in Patent Document 1) to generate a force in a direction parallel to the body surface. However, it is also possible to generate the force in a direction perpendicular to the body surface, as illustrated in **Fig. 6.** This is the same direction of the wave actuation of the conventionally used air cushions. Hence, the proposed turbine vibrator is compatible with the same MR-motion encoding sequences as used for conventionally used air cushions;

e) More accurate and faster control of the generated vibration force:

◦ As explained above, the rotational velocity of the turbine rotor and thus the generated force may be measured at higher temporal resolution and/or with reduced measurement times by detecting more than one optical signal during one rotation of the turbine rotor. This facilitates the control of the generated vibration force;

f) Better adaptability and control of the force generation over a broad force range:

◦ It is important, in particular for medical applications, to not only create a vibration large enough to obtain a sufficient signal to noise ratio, but also to be able to limit the vibration in the upper regime. For one, there are limits on the vibration (see EU whole-body vibration limit EU 2002/44/EC). For another, there is a limit of the vibration amplitude that is tolerable to the patient during the examination. This limit may vary for different body locations (e.g. head vs liver/lung vs thigh). Since a number of parameters of the proposed turbine vibrator (such as frequency and geometric requirements), may be appropriately selected and altered (individually or in combination), a better and more accurate control of the generated force can be achieved over a broad range of forces. Thus, optimizing the force generation around both the lower limit (to receive sufficient SNR) and around the upper limit can be more easily realized (with additional frequency and geometric requirements);

g) Compact design:

◦ Due to its compact design the passive driver may be placed at various locations of the elastic body to be examined and is easily set up and fixated to a patient using a body coil. Further, more than one passive driver can be placed on a patient for a multi-location multi-wave induction.

For example, it is possible to combine a plurality of turbine rotors for multi-location actuation.

For example, the passive driver may comprise a plurality of turbine vibrators connected in series to another through a common axle (common drive shaft). The active driver synchronously drives the turbine vibrators connected in series. **Fig.** 7 shows one example of connecting three turbine vibrators 10a, 10b and 10c in series though a common axle 19. The number of connected turbine vibrators is not restricted to three and may vary. The use of multiple synchronously driven mechanical actuators enables wave actuation at multiple locations on the body with synchronized mechanicals waves. This may increase the spatial resolution of the obtained elastograms, thereby enabling better detection of small inclusions, such as small tumors.

A number of embodiments have been described. Nevertheless, it will be understood that various modifications may be made. For example, the steps described can be performed in a different order and still achieve desirable results. Further, the pneumatic turbine may have other dimensions and/or configuration and may be subject to optimization for in-vivo imaging. Accordingly, other embodiments are within the scope of the claims.

**Reference numerals**

| 10, 10a-c | pneumatic turbine |
|---|---|
| 12 | adaptor plate |
| 14 | air inlet |
| 16 | housing |
| 17 | rotor chamber |
| 18 | turbine rotor (turbine wheel) |
| 19 | axle (shaft) |
| 20 | eccentric weight (unbalance) |
| 21 | bearings |
| 22 | sound absorber (air outlet) |
| 24 | body coil |
| 26 | patient |
| 28 | conduit (hose) |
| 30 | active driver (unit) |
| 32 | proportional pressure regulator |
| 33 | controllable resistance |

| 34 | sensor for detecting the actuation frequency |
| 36 | electro-optical interface |
| 37 | user interface (e.g. graphical user interface) |
| 38 | manual valve |
| 40 | pressure hose |
| 42 | phantom |
| 44 | inclusion |
| 50 | control room |
| 52 | scanner room |

## Claims

1. A mechanical actuator for Magnetic Resonance Elastography comprising:

   a passive driver including at least one rotational turbine vibrator (10) comprising a housing (16), a turbine rotor (18) arranged in the housing and an eccentric weight (20), said turbine vibrator (10) being powered by a fluid, and
   an active driver (30) configured to control the pressure of the fluid powering the turbine vibrator (10).

2. The mechanical actuator of claim 1, wherein the components constituting the passive driver are of non-magnetic and/or non-metallic material or materials.

3. The mechanical actuator of claim 2, wherein the housing (16), the turbine rotor (18) and the unbalance (20) are made of polymer and/or are 3D printed.

4. The mechanical actuator according to claim 3, wherein the polymer is any one of polyamide, polyurethanes, polypropylene, polycarbonate and acrylonitrile butadiene styrene.

5. The mechanical actuator of any one of the preceding claims, wherein the passive driver comprises an adaptor plate (12) connected to or merged with the rotational turbine vibrator (10), said adaptor plate configured to be positioned on and/or or fixed to a surface of a region of an elastic body to be examined.

6. The mechanical actuator according to any one of the preceding claims, wherein the generated centrifugal force is in the range of 0.1 to 50 N.

7. The mechanical actuator according to any one of the preceding claims, wherein the active driver (30) comprises a proportional pressure regulator (32) configured to control the pressure of the fluid powering the rotational turbine vibrator (10)

8. The mechanical actuator according to claim 7, wherein the proportional pressure regulator (32) is configured to stop the inflow of a fluid in the passive driver when (i) no control voltage is applied; and/or (ii) in case of power loss: and/or (iii) upon user input; and/or
   wherein the proportional pressure regulator (32) is configured to increase the control voltage gradually or stepwise to a predetermined value during a start-up phase; and/or
   wherein the active driver (30) comprises a manual valve (38) configured to stop the inflow of a fluid in the passive driver.

9. The mechanical actuator according to any one of the preceding claims, wherein the passive driver comprises a plurality of said rotational turbine vibrators (10a-c) connected in series through a common axle (19).

10. A magnetic resonance elastography system comprising the mechanical actuator according to any one of the preceding claims and a magnetic resonance imaging apparatus.

11. A method for inducing shear waves in an elastic body for magnetic resonance elastography comprising:

    providing a mechanical actuator according to any one of claims 1 to 9;
    positioning the passive driver (10) of the mechanical actuator on a region of the elastic body to be examined,
    controlling, by the active driver (30) of the mechanical actuator, the pressure of the compressed fluid powering the rotational turbine vibrator (10), thereby generating, by the rotational turbine vibrator (10), a vibration force having a predetermined frequency and/or magnitude.

12. A method according to claim 11, wherein the control voltage is adjusted to increase gradually or stepwise up to a predetermined value during a start-up phase; and/or
    wherein the inflow of fluid in the passive driver is stopped when no control voltage is applied and/or in case of power loss and/or upon user input.

13. A method for magnetic resonance elastography comprising:

    inducing shear waves in a region of an elastic body to be examined according to the method of any one of claims 11 or 12;
    imaging the propagation of the induced shear waves, thereby obtaining at least one wave image; and
    obtaining elasticity data of the region of the elastic body to be examined based on the at least one wave image.

14. A method for magnetic resonance elastography according to claim 13, wherein the shear waves are induced synchronously at a plurality of locations within said region of the elastic body to be examined.

15. A method for producing a mechanical actuator for Magnetic Resonance Elastography according to any one of claims 1 to 9, comprising producing the housing (16), the turbine rotor (18) and the unbalance (20) and/or a customized adaptor plate (12) by 3D printing.

**Fig. 1A**

**Fig. 1B**

EP 3 384 844 A1

**Fig. 1C**

**Fig. 2**

EP 3 384 844 A1

**Fig. 3**

**Fig. 4**

**Fig. 5**

**Fig. 6**

**Fig. 7**

Europäisches
Patentamt

European
Patent Office

Office européen
des brevets

# EUROPEAN SEARCH REPORT

Application Number

EP 17 00 0576

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 100 894 307 B1 (IND ACADEMIC COOP [KR]) 24 April 2009 (2009-04-24) * paragraphs [0001], [0024], [0032], [0039], [0040]; figures 1-5 * | 1-15 | INV. A61B5/055 B06B1/18 G01R33/563 |
| X | NICHOLAS J. CUTFIELD ET AL: "Visual and proprioceptive interaction in patients with bilateral vestibular loss", NEUROIMAGE: CLINICAL, vol. 4, 4 January 2014 (2014-01-04), pages 274-282, XP055295660, ISSN: 2213-1582, DOI: 10.1016/j.nicl.2013.12.013 | 1-9,15 | ADD. F03D9/00 |
| A | * section 2.4.2; figure 1A * | 10-14 | |
| A | H ELHAWARY ET AL: "A magnetic-resonance-compatible limb-positioning device to facilitate magic angle experiments in vivo", PROCEEDINGS OF THE INSTITUTION OF MECHANICAL ENGINEERS.JOURNAL OF ENGINEERING IN MEDICINE. PART H., vol. 222, no. 5, 16 July 2008 (2008-07-16), pages 751-760, XP55413893, GB ISSN: 0954-4119, DOI: 10.1243/09544119JEIM361 * the whole document * | 1-15 | |
| A,D | EP 2 499 970 A1 (UNIV TOKYO METROPOLITAN [JP]) 19 September 2012 (2012-09-19) * paragraphs [0022] - [0047]; figures 1-3 * | 1-15 | |

TECHNICAL FIELDS SEARCHED (IPC)

A61B
B06B
F03D
G01R

The present search report has been drawn up for all claims

| Place of search | Date of completion of the search | Examiner |
|---|---|---|
| The Hague | 13 October 2017 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 3 384 844 A1**

## ANNEX TO THE EUROPEAN SEARCH REPORT
## ON EUROPEAN PATENT APPLICATION NO.

EP 17 00 0576

13-10-2017

| Patent document cited in search report | | | Publication date | Patent family member(s) | | Publication date |
|---|---|---|---|---|---|---|
| KR 100894307 | B1 | | 24-04-2009 | NONE | | |
| EP 2499970 | A1 | | 19-09-2012 | EP | 2499970 A1 | 19-09-2012 |
| | | | | JP | 5376593 B2 | 25-12-2013 |
| | | | | JP | 2011098158 A | 19-05-2011 |
| | | | | US | 2011245658 A1 | 06-10-2011 |
| | | | | WO | 2011055469 A1 | 12-05-2011 |

EPO FORM P0459

For more details about this annex : see Official Journal of the European Patent Office, No. 12/82

**REFERENCES CITED IN THE DESCRIPTION**

**Patent documents cited in the description**

- EP 2499970 A1

**Non-patent literature cited in the description**

- **R. MUTHUPILLAI ; D. J. LOMAS ; P. J. ROSSMAN ; J. F. GREENLEAF ; A. MANDUCA ; R. L. EHMAN.** Magnetic resonance elastography by direct visualization of propagating acoustic strain waves. *Science,* 29 September 1995, vol. 269, 1854-7
- **R. S. SAHEBJAVAHER ; G. NIR ; M. HONARVAR ; L. O. GAGNON ; J. ISCHIA ; E. C. JONES et al.** MR elastography of prostate cancer: quantitative comparison with histopathology and repeatability of methods. *NMR Biomed,* January 2015, vol. 28, 124-39
- **A. MANDUCA ; T. E. OLIPHANT ; M. A. DRESNER ; J. L. MAHOWALD ; S. A. KRUSE ; E. AMROMIN et al.** Magnetic resonance elastography: non- invasive mapping of tissue elasticity. *Med Image Anal,* December 2001, vol. 5, 237-54
- **A. ARANI ; K. L. GLASER ; S. P. ARUNACHALAM ; P. J. ROSSMAN ; D. S. LAKE ; J. D. TRZASKO et al.** In vivo, high-frequency three- dimensional cardiac MR elastography: Feasibility in normal volunteers. *Magnetic Resonance in Medicine,* 2017, vol. 77, 351-360
- **R. S. SAHEBJAVAHER ; A. BAGHANI ; M. HONARVAR ; R. SINKUS ; S. E. SALCUDEAN.** Transperineal prostate MR elastography: initial in vivo results. *Magn. Reson. Med.,* February 2013, vol. 69, 411-20
- **R. CHOPRA ; A. ARANI ; Y. HUANG ; M. MUSQUERA ; J. WACHSMUTH ; M. BRONSKILL et al.** In vivo MR elastography of the prostate gland using a transurethral actuator. *Magn. Reson. Med.,* September 2009, vol. 62, 665-71
- **S. K. VENKATESH ; R. L. EHMAN.** Magnetic resonance elastography of abdomen. *Abdom Imaging,* April 2015, vol. 40, 745-59

- **M. YIN ; J. CHEN ; K. J. GLASER ; J. A. TALWALKAR ; R. L. EHMAN.** Abdominal magnetic resonance elastography. *Top Magn. Reson. Imaging,* April 2009, vol. 20, 79-87
- **O. ROUVIERE ; M. YIN ; M. A. DRESNER ; P. J. ROSSMAN ; L. J. BURGART ; J. L. FIDLER et al.** MR elastography of the liver: preliminary results. *Radiology,* August 2006, vol. 240, 440-8
- **M. YIN ; J. A. TALWALKAR ; K. J. GLASER ; A. MANDUCA ; R. C. GRIMM ; P. J. ROSSMAN et al.** Assessment of hepatic fibrosis with magnetic resonance elastography. *Clin Gastroenterol Hepatol,* October 2007, vol. 5, 1207-1213 e2
- **S. K. VENKATESH ; M. YIN ; J. F. GLOCKNER ; N. TAKAHASHI ; P. A. ARAOZ ; J. A. TALWALKAR et al.** MR elastography of liver tumors: preliminary results. *AJR Am J Roentgenol,* June 2008, vol. 190, 1534-40
- **P. LATTA ; M. L. GRUWEL ; P. DEBERGUE ; B. MATWIY ; U. N. SBOTO- FRANKENSTEIN ; B. TOMANEK.** Convertible pneumatic actuator for magnetic resonance elastography of the brain. *Magn. Reson. Imaging,* January 2011, vol. 29, 147-52
- **L. HUWART ; F. PEETERS ; R. SINKUS ; L. ANNET ; N. SALAMEH ; L. C. TERBEEK et al.** Liver fibrosis: non-invasive assessment with MR elastography. *NMR Biomed,* April 2006, vol. 19, 173-9
- **K. UFFMANN ; C. ABICHT ; W. GROTE ; H. H. QUICK ; M. E. LADD.** Concepts in Magnetic Resonance. *Design of an MR-compatible piezoelectric actuator for MR elastography,* 2002, vol. 15, 239-254
- **K. UFFMANN ; M. E. LADD.** Actuation systems for MR elastography: design and applications. *IEEE Eng. Med. Biol. Mag.,* May 2008, vol. 27, 28-34